# EUROPEAN PATENT SPECIFICATION

(11) **EP 2 916 134 B1**
(45) Date of publication and mention of the grant of the patent: **16.08.2017**
(21) Application number: 14157822.9
(22) Date of filing: 05.03.2014
(51) Int. Cl.: G01N 33/68

(54) **Use of Seprase for differential diagnosis of acute dyspnea**
Verwendung von Seprase zur Differenzialdiagnose von akuter Atemnot
Utilisation de la séprase pour le diagnostic différentiel de la dyspnée aiguë

(43) Date of publication of application: 09.09.2015
(73) Proprietor: Roche Diagnostics GmbH, 68305 Mannheim (DE); F. Hoffmann-La Roche AG, 4070 Basel (CH)
(72) Inventor: Rollinger, Wolfgang, 86935 Rott (DE); Karl, Johann, 82380 Peissenberg (DE); Roeddiger, Ralf, D- 69517 Gorxheimertal (DE); Swiatek-De Lange, Magdalena, 82377 Penzberg (DE); Ehret, Christoph, 81477 München (DE)
(74) Representative: Herzog, Fiesser & Partner Patentanwälte PartG mbB

(56) References cited:
- EP-A1- 2 031 397
- WO-A1-2009/074275
- WO-A1-2012/123293
- US-A1- 2012 021 929
- FIONA M. KEANE ET AL: "Neuropeptide?Y, B-type natriuretic peptide, substance?P and peptide?YY are novel substrates of fibroblast activation protein-[alpha]", FEBS JOURNAL, vol. 278, no. 8, 9 March 2011 (2011-03-09) , pages 1316-1332, XP55131799, ISSN: 1742-464X, DOI: 10.1111/j.1742-4658.2011.08051.x

## Description

The present invention relates to a method for differentiating in a patient who suffers from acute shortness of breath (acute dyspnea) between pulmonary disease and cardiac disease. The method is based on measuring the levels of seprase and of a cardiac marker in a sample from said patient. Further envisaged are kits and devices adapted to carry out the method of the present invention.

Cardiac diseases and, in particular, acute cardiovascular events are most often life threatened medical conditions which require immediate action. However, these conditions can not always be unambiguously diagnosed. Specifically, some of the most common symptoms accompanying various types of heart diseases including acute cardiovascular events but also chronic heart dysfunctions such as chronic heart failure are symptoms which are characteristic for other (non-cardiac) diseases as well. Therefore, it is often difficult, cumbersome and time consuming to differentiate between a cardiac or other cause of an observed symptom. Said differentiation may also require the help of a specialist such as a cardiologist.

A typical symptom for a cardiac disease, in particular, for an acute cardiovascular event or a more severe chronic heart failure is shortness of breath (dyspnea). As for other symptoms, dyspnea may have various causes including cardiac complications and non-cardiac pulmonary diseases. In light of a potential cardiac cause of the symptom, it is highly advisable to properly diagnose its cause in a given patient, e.g., an emergency patient.

Diagnosis of dyspnea is mostly performed using electrocardiogram and chest radiographs. Some dyspnea biomarker panels do not appear to improve accuracy of differential diagnosis.

NT-proBNP has been also described together with seven additional parameters as an indicator for acute heart failure in patients exhibiting dyspnoea (Baggish 2005, American Heart Journal, 151:48-54, Januzzi JL et.al. International Collaborative ofNTproBNP Study European Heart Journal 2006; 27; 330-337).

EP 1 845 380 A1, EP 1 845 380 A1 and WO02009/027339 disclose the use of surfactant proteins SP-B and SP-D in differentiating the causes of shortness of breath.

Recently, the enzyme Seprase has turned out to be an important marker for early diagnose of a colorectal cancer. Human Seprase is a 170 kD, single-pass membrane-bound glycoprotein having gelatinase and dipeptidyl peptidase activity and consists of two identical monomeric units. Seprase is essentially identical to fibroblast activation protein (FAP-alpha). It has been shown that FAP-alpha is strongly expressed in the malignant of bone and soft tissue sarcomas. Since 1990 it has also been known that most of the common types of epithelial cancers, including breast, lung, skin, pancreas and CRC, contain abundant FAP-alpha-positive stromal fibroblasts.

WO2009/074275 discloses the use of the human fibroblast activation protein (FAP/seprase) as a marker of cancer particularly of pulmonary or lung cancer (=LC) or of colon cancer and most particularly in the assessment of non-small cell lung carcinoma (NSCLC) or colorectal cancer. Furthermore, it especially relates to a method for assessing cancer from a liquid sample, derived from an individual by measuring seprase in said sample. Measurement of seprase can, e.g., be used in the early detection of cancer or in the surveillance of patients who undergo surgery.

WO2010/127782 discloses the use of "soluble DPPIV/Seprase protein complex" (= DPPIV/Seprase) as a universal marker of different cancer types. Measurement of DPPIV/Seprase can, e.g., be used in the early detection or diagnosis of cancer or in the surveillance of patients who undergo surgery.

WO 2012/123293 describes the use of the protein Seprase as a marker in the assessment of chronic obstructive pulmonary disease (= COPD).

WO2009/074276 relates to a method for assessing colorectal cancer (CRC) by measuring the concentration and/or activity of a seprase polypeptide and/or fragments thereof and further biomarker as anti-p53, osteopontin, Ferritin, e.g. in the early detection of cancer by screening of asymptomatic individuals or in the surveillance of patients who undergo surgery.

There is a clear long-standing need for means and methods allowing a differential diagnosis of the cause of acute dyspnea in a subject. The said means and methods shall allow a reliable efficient diagnosis and shall avoid the drawbacks of the current techniques.

Thus, the technical problem underlying the present invention must be seen as the provision of means and methods for complying with the aforementioned needs.

The technical problem is solved by the embodiments characterized in the claims and herein below.

Thus, the technical problem underlying the present invention must be seen as the provision of means and methods for complying with the aforementioned needs.

The technical problem is solved by the embodiments characterized in the claims and herein below.

Accordingly, the present invention relates to a method for differentiating in a patient who suffers from acute shortness of breath (acute dyspnea) between pulmonary disease and cardiac disease, said method comprising the steps of
a) measuring a level of seprase in a sample from said patient,
b) measuring a level of a cardiac marker in a sample from said patient, wherein the cardiac marker is NT-proBNP or BNP
c) comparing the level as measured in step a) to a reference level, and
d) comparing the level as measured in step b) to a reference level.

In an embodiment, steps a) and b) are carried out by contacting the sample with an agent that specifically binds to the seprase (step a)) and to the cardiac marker (step b)), respectively, thereby forming a complex between the agent and the seprase and the cardiac marker, respectively, detecting the amount of complex formed between the agent and the seprase and the amount of complex formed between the agent and the cardiac marker, thereby measuring the levels of the seprase and the cardiac marker, respectively.

In an embodiment, the aforementioned method may comprise step e) of differentiating (or providing a differentiation) between pulmonary disease and cardiac disease based on the results of the comparison steps c) and d).

The term "differentiating between pulmonary disease and cardiac disease" is used to indicate that the method according to the present invention will aid a medical professional including, e.g., a physician in assessing whether an individual who suffers from acute shortness of breath between pulmonary disease and cardiac disease as underlying causes for said acute shortness of breath. The term "differentiating" as used herein, in particular, means to distinguish between a subject who suffers from pulmonary disease and/or cardiac disease (in particular from pulmonary disease and/or cardiac disease as cause for acute shortness of breath) under conditions where the subjects suffering from said disease show essentially the same symptoms, i.e. acute shortness of breath. Thus, the method envisaged the diagnosis of pulmonary disease and/or cardiac disease in a patient who suffers from acute shortness of breath. The term as used herein, preferably, includes differentially diagnosing a pulmonary disease or cardiac disease.

The phrase "providing a differentiation" as used herein refers to using the information or data generated relating to the levels of seprase and a cardiac marker in a sample of a patient to differentiate between pulmonary disease and cardiac disease in a patient who suffers from acute shortness of breath. The information or data may be in any form, written, oral or electronic. In some embodiments, using the information or data generated includes communicating, presenting, reporting, storing, sending, transferring, supplying, transmitting, dispensing, or combinations thereof. In some embodiments, communicating, presenting, reporting, storing, sending, transferring, supplying, transmitting, dispensing, or combinations thereof are performed by a computing device, analyzer unit or combination thereof. In some further embodiments, communicating, presenting, reporting, storing, sending, transferring, supplying, transmitting, dispensing, or combinations thereof are performed by a laboratory or medical professional. In some embodiments, the information or data includes a comparison of the levels as measured in steps a) and b) to reference levels (in steps c) and d)). In some embodiments, the information or data includes an indication that the patient suffers from pulmonary disease or cardiac disease.

The expression "acute shortness of breath" or "acute dyspnea" refers to an impaired respiration which results in an increased respiratory frequency and/or an increased respiratory volume. Thus, shortness of breath may result, preferably, in hyperventilation. Shortness of breath occurs, usually, at an oxygen saturation level below the normal oxygen saturation level of at least 95%. Acute dyspnea as used herein refers to a non-permanently occurring shortness of breath, i. e. shortness of breath which occurs all over sudden (acute onset dyspnea) and which is not correlated to a specific condition, e. g., which occurs only under certain types of stress etc.. Moreover, acute dyspnea preferably persists no longer than 2 weeks, in particular no longer than one week from the acute onset, while chronic dyspnea is characterized as persisting for a period of time longer than 2 weeks, or in particular longer than 1 month. Furthermore, acute dyspnea is, usually, progressively worsening.

The term "pulmonary disease" refers to any lung disease which causes acute shortness of breath. Moreover, it is envisaged that a pulmonary disease as referred to in accordance with the present invention shall result in an impaired alveolocapillary membrane barrier. Said disease is, preferably, acute and chronic respiratory failure, COPD, asthma, pneumothorax, pulmonary infection, ARDS (acute respiratory distress syndrome), lung injury or hemorrhage, pulmonary fibrosis, pulmonary proteinosis, pulmonary oedema, pulmonary inflammation, pulmonary emphysema obesity, thyroid diseases or, more preferably, a pulmonary embolism. In particular, the pulmonary disease is COPD, asthma, pneumothorax, pulmonary infection, ARDS, lung injury or hemorrhage, or pulmonary embolism.

The term "cardiac disease" as used herein refers to any acute or chronic disorder of the cardiovascular system. Acute disorders of the cardiovascular system include acute cardiovascular events. Thus, more preferably, encompassed are stable angina pectoris (SAP) or acute coronary syndromes (ACS). ACS patients can show unstable angina pectoris (UAP) or these individuals have already suffered from a myocardial infarction (MI). MI can be an ST-elevated MI or a non-ST-elevated MI. The occurring of an MI can be followed by a left ventricular dysfunction (LVD). Also encompassed by the term are chronic disorders and, preferably, heart failure such as left ventricular or right ventricular heart failure. It is to be understood that the term also includes medical conditions and diseases which cause heart failure in addition to the aforementioned acute cardiovascular events, such as congenital or acquired heart valve diseases or disorders, myocarditis, myocardiopathy, amyloidosis or hemochromatosis. Further preferred cardiac diseases are thrombosis, preferably arterial thrombosis, or diseases causing blood vessel calcification, preferably atherosclerosis, as well as stroke. In particular, the cardiac disease may be congestive heart failure, ACS, cardiomyopathy, valvular dysfunction, arrhythmia, and acutely decompensated heart failure.

The individuals suffering from a cardiac disease may show clinical symptoms (e.g. dyspnea, chest pain, see also NYHA classification below). Specifically, symptoms of cardiac diseases have been classified into a functional classification system according to the New York Heart Association (NYHA). Patients of Class I have no obvious symptoms of cardiac disease. Physical activity is not limited, and ordinary physical activity does not cause undue fatigue, palpitation, or dyspnea. Patients of class II have slight limitation of physical activity. They are comfortable at rest, but ordinary physical activity results in fatigue, palpitation, or dyspnea. Patients of class III show a marked limitation of physical activity. They are comfortable at rest, but less than ordinary activity causes fatigue, palpitation, or dyspnea. Patients of class IV are unable to carry out any physical activity without discomfort. They show symptoms of cardiac insufficiency at rest. If any physical activity is undertaken, discomfort is increased. Another characteristic of cardiac complication can be the "left ventricular ejection fraction" (LVEF) which is also known as "ejection fraction". People with a healthy heart usually have an unimpaired LVEF, which is generally described as above 50 %. Most people with a systolic heart disease which is symptomatic generally have an LVEF of 40 % or less.

Preferably, a subject suffering from a cardiac disease and exhibiting acute dyspnea in accordance with the present invention can be allocated to an intermediated NYHA class, preferably, to NYHA class I, II or III and, most preferably, to NYHA class II.

The "subject" or "patient" as referred to herein is, preferably, a mammal. Mammals include, but are not limited to, domesticated animals (e.g., cows, sheep, cats, dogs, and horses), primates (e.g., humans and non-human primates such as monkeys), rabbits, and rodents (e.g., mice and rats). Preferably, the patient is a human patient. The terms "subject" and "patient" may be used interchangeably herein.

In a preferred embodiment of the present invention, it is envisaged that the patient does not suffer from cancer.

The term "cancer", as used herein, preferably refers to a proliferative disorder disease caused or characterized by the proliferation of cells which have lost susceptibility to normal growth control. The term, preferably, includes solid tumors arising in solid tissues or organs as well as hematopoietic tumors. The term encompasses tumors and any other proliferative disorders. Thus, the term is meant to include all pathological conditions involving malignant cells, irrespective of stage or of invasiveness. Furthermore, it is particularly contemplated that the cancer invasive, and, thus may have spread beyond the layer of tissue in which it originated into the normal surrounding tissues (frequently also referred to as locally advanced cancer). Invasive cancers may or not be metastatic. A cancer is metastatic, if it has spread from its original location to distant parts of the body. It is particularly contemplated that the term "cancer" refers to metastatic cancer. Accordingly, the subject in the context of the aforementioned method, preferably, does not suffer from metastatic cancer.

The cancer may be localized to a specific tissue or organ (e.g. in the breast, the prostate or the lung), and, thus, may not have spread beyond the tissue of origin.

Moreover, it is also envisaged that the cancer is selected from the group consisting of cervical cancer, colorectal cancer, gastrointestinal cancer, leukaemia, lung cancer, mesothelioma, non-hodgkin's lymphoma, non-small cell lung cancer, ovarian cancer, pancreatic cancer, prostate cancer, skin cancer, small cell lung cancer, brain tumors, uterine cancer, head and neck tumors, liver cancer, renal cancer, neuroblastoma, intestine carcinoma such as rectum carcinoma, familiary adenomatous polyposis carcinoma and hereditary non-polyposis, esophageal carcinoma, labial carcinoma, larynx carcinoma, hypopharynx carcinoma, tong carcinoma, salivary gland carcinoma, adenocarcinoma, medullary thyroidea carcinoma, papillary thyroidea carcinoma, renal carcinoma, kidney parenchym carcinoma, ovarian carcinoma, cervical carcinoma, uterine corpus carcinoma, endometriuni carcinoma, chorion carcinoma, pancreatic carcinoma, testis carcinoma, urinary carcinoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, small cell lung carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma, plasmocytoma, Kaposi's sarcoma and melanoma.

The term "sample" refers to a sample of a body fluid, to a sample of separated cells or to a sample from a tissue or an organ. Samples of body fluids can be obtained by well-known techniques and include, samples of blood, plasma, serum, urine, lymphatic fluid, sputum, ascites, or any other bodily secretion or derivative thereof. Tissue or organ samples may be obtained from any tissue or organ by, e.g., biopsy. Separated cells may be obtained from the body fluids or the tissues or organs by separating techniques such as centrifugation or cell sorting. E.g., cell-, tissue- or organ samples may be obtained from those cells, tissues or organs which express or produce the biomarker. The sample may be frozen, fresh, fixed (e.g. formalin fixed), centrifuged, and/or embedded (e.g. paraffin embedded), etc. The cell sample can, of course, be subjected to a variety of well-known post-collection preparative and storage techniques (e.g., nucleic acid and/or protein extraction, fixation, storage, freezing, ultrafiltration, concentration, evaporation, centrifugation, etc.) prior to assessing the level of the marker in the sample. Likewise, biopsies may also be subjected to post-collection preparative and storage techniques, e.g., fixation.

In a preferred embodiment, the sample is a blood, plasma or, in particular, a serum sample.

The term "measuring" the level of a marker as referred to herein refers to the quantification of the biomarker, e.g. to determining the level of the biomarker in the sample, employing appropriate methods of detection described elsewhere herein.

In an embodiment, the level of the at least one biomarker is measured by contacting the sample with a detection agent that specifically binds to the respective marker, thereby forming a complex between the agent and said marker, detecting the level of complex formed, and thereby measuring the level of said marker.

The biomarkers as referred to herein can be detected using methods generally known in the art. Methods of detection generally encompass methods to quantify the level of a biomarker in the sample (quantitative method). It is generally known to the skilled artisan which of the following methods are suitable for qualitative and/or for quantitative detection of a biomarker. Samples can be conveniently assayed for, e.g., proteins using Westerns and immunoassays, like ELISAs, RIAs, fluorescence-based immunoassays, which are commercially available. Further suitable methods to detect biomarker include measuring a physical or chemical property specific for the peptide or polypeptide such as its precise molecular mass or NMR spectrum. Said methods comprise, e.g., biosensors, optical devices coupled to immunoassays, biochips, analytical devices such as mass- spectrometers, NMR- analyzers, or chromatography devices. Further, methods include microplate ELISA-based methods, fully-automated or robotic immunoassays (available for example on El-ecsysTM analyzers), CBA (an enzymatic Cobalt Binding Assay, available for example on Roche-HitachiTM analyzers), and latex agglutination assays (available for example on Roche-HitachiTM analyzers).

For the detection of biomarker proteins as referred to herein a wide range of immunoassay techniques using such an assay format are available, see, e.g., U.S. Pat. Nos. 4,016,043, 4,424,279, and 4,018,653. These include both single-site and two-site or "sandwich" assays of the non-competitive types, as well as in the traditional competitive binding assays. These assays also include direct binding of a labeled antibody to a target biomarker.

Sandwich assays are among the most useful and commonly used immunoassays.

Methods for measuring electrochemiluminescent phenomena are well-known. Such methods make use of the ability of special metal complexes to achieve, by means of oxidation, an excited state from which they decay to ground state, emitting electrochemiluminescence. For review see Richter, M.M., Chem. Rev. 104 (2004) 3003-3036.

Biomarkers can also be detected by generally known methods including magnetic resonance spectroscopy (NMR spectroscopy), Gas chromatography-mass spectrometry (GC-MS), Liquid chromatography-mass spectrometry (LC-MS), High and ultra-HPLC HPLC such as reverse phase HPLC, for example, ion-pairing HPLC with dual UV-wavelength detection, capillary electrophoresis with laser-induced fluorescence detection, anion exchange chromatography and fluorescent detection, thin layer chromatography.

Preferably, measuring the level of a biomarker as referred to herein comprises the steps of (a) contacting a cell capable of eliciting a cellular response the intensity of which is indicative of the level of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the level of the peptide or polypeptide.

Also preferably, measuring the level of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Measuring the level of a peptide or polypeptide may, preferably, comprises the steps of (a) contacting the peptide with a specific binding agent, (b) (optionally) removing non-bound binding agent, (c) measuring the level of bound binding agent, i.e. the complex of the binding agent formed in step(a). According to a preferred embodiment, said steps of contacting, removing and measuring may be performed by an analyzer unit of the system disclosed herein. According to some embodiments, said steps may be performed by a single analyzer unit of said system or by more than one analyzer unit in operable communication with each other. For example, according to a specific embodiment, said system disclosed herein may include a first analyzer unit for performing said steps of contacting and removing and a second analyzer unit, operably connected to said first analyzer unit by a transport unit (for example, a robotic arm), which performs said step of measuring.

The bound binding agent, i.e. the binding agent or the binding agent/peptide complex, will generate an intensity signal. Binding according to the present invention includes both covalent and non-covalent binding. A binding agent according to the present invention can be any compound, e.g., a peptide, polypeptide, nucleic acid, or small molecule, binding to the peptide or polypeptide described herein. Preferred binding agents include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such binding agents are well-known in the art. For example, identification and production of suitable antibodies or aptamers is also offered by commercial suppliers. The person skilled in the art is familiar with methods to develop derivatives of such binding agents with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the binding agent or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the binding agent can be measured by any method known in the art. Preferably, said method is semi-quantitative or quantitative. Further suitable techniques for the determination of a polypeptide or peptide are described in the following.

Binding of a binding agent may be measured directly, e.g. by NMR or surface plasmon resonance. Measurement of the binding of a binding agent, according to preferred embodiments, is performed by an analyzer unit of a system disclosed herein. Thereafter, a level of the measured binding may be calculated by a computing device of a system disclosed herein. If the binding agent also serves as a substrate of an enzymatic activity of the peptide or polypeptide of interest, an enzymatic reaction product may be measured (e.g. the level of a protease can be measured by measuring the level of cleaved substrate, e.g. on a Western Blot). Alternatively, the binding agent may exhibit enzymatic properties itself and the "binding agent/peptide or polypeptide" complex or the binding agent which was bound by the peptide or polypeptide, respectively, may be contacted with a suitable substrate allowing detection by the generation of an intensity signal. For measurement of enzymatic reaction products, preferably the level of substrate is saturating. The substrate may also be labeled with a detectable label prior to the reaction. Preferably, the sample is contacted with the substrate for an adequate period of time. An adequate period of time refers to the time necessary for a detectable, preferably measurable, level of product to be produced. Instead of measuring the level of product, the time necessary for appearance of a given (e.g. detectable) level of product can be measured. Third, the binding agent may be coupled covalently or non-covalently to a label allowing detection and measurement of the binding agent. Labeling may be done by direct or indirect methods. Direct labeling involves coupling of the label directly (covalently or non-covalently) to the binding agent. Indirect labeling involves binding (covalently or non-covalently) of a secondary binding agent to the first binding agent. The secondary binding agent should specifically bind to the first binding agent. Said secondary binding agent may be coupled with a suitable label and/or be the target (receptor) of tertiary binding agent binding to the secondary binding agent. The use of secondary, tertiary or even higher order binding agents is often used to increase the signal. Suitable secondary and higher order binding agents may include antibodies, secondary antibodies, and the well-known streptavidin-biotin system (Vector Laboratories, Inc.). The binding agent or substrate may also be "tagged" with one or more tags as known in the art. Such tags may then be targets for higher order binding agents. Suitable tags include biotin, digoxygenin, His-Tag, Glutathion-S-Transferase, FLAG, GFP, myc-tag, influenza A virus hae-magglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, avail-able as ready-made stock solution from Roche Diagnostics), CDP-Star™ (Amersham Bio-sciences), ECF™ (Amersham Biosciences). A suitable enzyme-substrate combination may result in a colored reaction product, fluorescence or chemoluminescence, which can be measured according to methods known in the art (e.g. using a light-sensitive film or a suit-able camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. A radioactive label can be detected by any method known and appropriate, e.g. a light-sensitive film or a phosphor imager.

The level of a peptide or polypeptide may be, also preferably, determined as follows: (a) contacting a solid support comprising a binding agent for the peptide or polypeptide as specified above with a sample comprising the peptide or polypeptide and (b) measuring the level peptide or polypeptide which is bound to the support. The binding agent, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The binding agent or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said binding agent are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different binding agents. Methods of producing such arrays, for example based on solid-phase chemistry and photo-labile protective groups, are generally known (US 5,744,305).

In an embodiment of the present invention, the levels of the biomarkers as referred to herein are measured by using the assays described in the Examples section.

In another embodiment of the method of the present invention, the measurement in step a) and b) may be carried out by an analyzer unit, in particular by an analyzer unit as defined elsewhere herein.

The term "binding agent" or "detection agent" refers to a molecule that comprises a binding moiety which specifically binds the corresponding to the respective biomarker. Examples of "binding agent" or "detection agent" are a aptamer, antibody, antibody fragment, peptide, peptide nucleic acid (PNA) or chemical compound.

The term "specific binding" or "specifically bind" refers to a binding reaction wherein binding pair molecules exhibit a binding to each other under conditions where they do not significantly bind to other molecules.

The term "specific binding" or "specifically binds", when referring to a protein or peptide as a binding agent, refers to a binding reaction wherein a binding agent binds to the corresponding target molecule with an affinity of at least 10⁻⁷ M. The term "specific binding" or "specifically binds" preferably refers to an affinity of at least 10⁻⁸ M or even more preferred of at least 10⁻⁹ M for its target molecule. The term "specific" or "specifically" is used to indicate that other molecules present in the sample do not significantly bind to the binding agent specific for the target molecule. Preferably, the level of binding to a molecule other than the target molecule results in a binding affinity which is only 10% or less, more preferably only 5% or less of the affinity to the target molecule.

The term "specific binding" or "specifically binds", when referring to a nucleic acid as a binding agent, refers to a hybridization reaction wherein a binding agent or a probe contains a hybridizing region exactly or substantially complementary to the target sequence of interest. A hybridization assay carried out using the binding agent or probe under sufficiently stringent hybridization conditions enables the selective detection of the specific target sequence. The hybridizing region is preferably from about 10 to about 35 nucleotides in length, more preferably from about 15 to about 35 nucleotides in length. The use of modified bases or base analogues which affect the hybridization stability, which are well known in the art, may enable the use of shorter or longer probes with comparable stability. A binding agent or a probe can either consist entirely of the hybridizing region or can contain additional features which allow for the detection or immobilization of the probe, but which do not significantly alter the hybridization characteristics of the hybridizing region. The term "specific binding" or "specifically binds", when referring to a nucleic acid aptamer as a binding agent, refers to a binding reaction wherein a nucleic acid aptamer binds to the corresponding target molecule with an affinity in the low nM to pM range.

Examples of "binding agents", "detection agents" or "agents" are a nucleic acid probe, nucleic acid primer, DNA molecule, RNA molecule, aptamer, antibody, antibody fragment, peptide, peptide nucleic acid (PNA) or chemical compound. A preferred agent is an antibody which specifically binds to the biomarker to be measured. The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity. Preferably, the antibody is a polyclonal antibody. More preferably, the antibody is a monoclonal antibody.

Another binding agent that can be applied, in an aspect, may be an aptamere which specifically binds to the at least one marker in the sample. The term "specific binding" or "specifically binds", when referring to a nucleic acid aptamer as a binding agent, refers to a binding reaction wherein a nucleic acid aptamer binds to the corresponding target molecule with an affinity in the low nM to pM range.

In yet an aspect the, sample is removed from the complex formed between the binding agent and the at least one marker prior to the measurement of the level of formed complex. Accordingly, in an aspect, the binding agent may be immobilized on a solid support. In yet an aspect, the sample can be removed from the formed complex on the solid support by applying a washing solution. The formed complex shall be proportional to the level of the at least one marker present in the sample. It will be understood that the specificity and/or sensitivity of the binding agent to be applied defines the degree of proportion of at least one marker comprised in the sample which is capable of being specifically bound. Further details on how the determination can be carried out are also found elsewhere herein. The level of formed complex shall be transformed into a level of at least one marker reflecting the level indeed present in the sample. Such a level, in an aspect, may be essentially the level present in the sample or may be, in another aspect, an level which is a certain proportion thereof due to the relationship between the formed complex and the level present in the original sample.

In accordance with present invention, it is contemplated to measure the level of two markers, i..e. of seprase and of NT-proBNP or BNP. Natriuretic peptides are well known in the art. The term "natriuretic peptide" comprises Atrial Natriuretic Peptide (ANP)-type and Brain Natriuretic Peptide (BNP)-type peptides and variants thereof having the same predictive potential. Natriuretic peptides according to the present invention comprise ANP-type and BNP-type peptides and variants thereof (see e.g. Bonow, R.O. (1996). New insights into the cardiac natriuretic peptides. Circulation 93: 1946-1950). ANP-type peptides comprise pre-proANP, proANP, NT-proANP, and ANP. BNP-type peptides comprise pre-proBNP, proBNP, NT-proBNP, and BNP. In particular, the BNP-type peptide is NT-proBNP or BNP.

The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). Preferred natriuretic peptides according to the present invention are NT-proANP, ANP, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NT-proBNP is 120 min longer than that of BNP, which is 20 min (Smith MW, Espiner EA, Yandle TG, Charles CJ, Richards AM. Delayed metabolism of human brain natriuretic peptide reflects resistance to neutral endopeptidase. J Endocrinol. 2000; 167: 239-46).

Preanalytics are more robust with NT-proBNP allowing easy transportation of the sample to a central laboratory (Mueller T, Gegenhuber A, Dieplinger B, Poelz W, Haltmayer M. Long-term stability of endogenous B-type natriuretic peptide (BNP) and amino terminal proBNP (NT-proBNP) in frozen plasma samples. Clin Chem Lab Med 2004; 42: 942-4). Blood samples can be stored at room temperature for several days or may be mailed or shipped without recovery loss. In contrast, storage of BNP for 48 hours at room temperature or at 4 deg. Celsius leads to a concentration loss of at least 20 % (Mueller T, Gegenhuber A, et al., Clin Chem Lab Med 2004; 42: 942-4 , supra; Wu AH, Packer M, Smith A, Bijou R, Fink D, Mair J, Wallentin L, Johnston N, Feldcamp CS, Haverstick DM, Ahnadi CE, Grant A, Despres N, Bluestein B, Ghani F. Analytical and clinical evaluation of the Bayer ADVIA Centaur automated B-type natriuretic peptide assay in patients with heart failure: a multisite study. Clin Chem 2004; 50: 867-73). Therefore, depending on the time-course or properties of interest, either measurement of the active or the inactive forms of the natriuretic peptide can be advantageous.

The most preferred natriuretic peptides according to the present invention are NT-proBNP or variants thereof. As briefly discussed above, the human NT-proBNP as referred to in accordance with the present invention is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913, Bonow 1996, New Insights into the cardiac natriuretic peptides. Circulation 93: 1946-1950. Preferably, human NT-proBNP as used herein is human NT-proBNP as disclosed in EP 0 648 228 B1.

The NT-proBNP referred to in accordance with the present invention further encompasses allelic and other variants of said specific sequence for human NT-proBNP discussed above. Specifically, envisaged are variant polypeptides which are on the amino acid level at least 60 % identical, more preferably at least 70 %, at least 80 %, at least 90 %, at least 95 %, at least 98% or at least 99 % identical, to human NT-proBNP, in particular over the entire length. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be specifically recognizable (i. e. without cross reactions) by the aforementioned means or ligands used for determination of the amount of the natriuretic peptides. Biological and/or immunological NT-proBNP properties can be detected by the assay described in Karl et al. (Karl 1999. Development of a novel, N-Terminal-proBNP (NT-proBNP) assay with a low detection limit. Scand J Clin Invest 230:177-181), Yeo et al. (Yeo 2003. Multicenter evaluation of the Roche NT-proBNP assay and comparison to the Biosite Triage assay. Clinica Chimica Acta 338:107-115), and in the Example, below. Variants also include posttranslationally modified natriuretic peptides such as glycosylated peptides. A variant in accordance with the present invention is also a peptide or polypeptide which has been modified after collection of the sample, for example by covalent or non-covalent attachment of a label, particularly a radioactive or fluorescent label, to the peptide.

The term "cardiac Troponin" refers to all Troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac Troponin refers to Troponin T and/or Troponin I, and, most preferably, to Troponin T. It is to be understood that isoforms of Troponins may be measured in the method of the present invention together, i.e. simultaneously or sequentially, or individually, i.e. without measuring the other isoform at all. Amino acid sequences for human Troponin T and human Troponin I are disclosed in Anderson, loc cit and Ferrieres 1998, Clinical Chemistry, 44: 487-493.

The term "cardiac Troponin" encompasses also variants of the aforementioned specific Troponins, i.e., preferably, of Troponin I, and more preferably, of Troponin T. Such variants have at least the same essential biological and immunological properties as the specific cardiac Troponins. In particular, they share the same essential biological and immunological properties if they are detectable by the same specific assays referred to in this specification, e.g., by ELISA Assays using polyclonal or monoclonal antibodies specifically recognizing the said cardiac Troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the specific Troponin (in particular over the entire length). Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants may be allelic variants or any other species specific homologs, paralogs, or orthologs. Moreover, the variants referred to herein include fragments of the specific cardiac Troponins or the aforementioned types of variants as long as these fragments have the essential immunological and biological properties as referred to above. Preferably, the cardiac troponin variants have immunological properties (i.e. epitope composition) comparable to those of human troponin T or troponin I. Thus, the variants shall be recognizable by the aforementioned means or ligands used for determination of the concentration of the cardiac troponins. Thus, the variants shall be recognizable by the aforementioned means or ligands used for determination of the concentration of the cardiac troponins. Such fragments may be, e.g., degradation products of the Troponins. Further included are variants which differ due to posttranslational modifications such as phosphorylation or myristylation. Preferably the biological property of troponin I and its variant is the ability to inhibit actomyosin ATPase or to inhibit angiogenesis in vivo and in vitro, which may e.g. be detected based on the assay described by Moses et al. 1999 PNAS USA 96 (6): 2645-2650). Preferably the biological property of troponin T and its variant is the ability to form a complex with troponin C and I, to bind calcium ions or to bind to tropomyosin, preferably if present as a complex of troponin C, I and T or a complex formed by troponin C, troponin I and a variant of troponin T. It is known that low concentrations of circulating cardiac troponin may be detected in subjects at various conditions, but further studies are required to understand their respective role and rate (Masson et al., Curr Heart Fail Rep (2010) 7:15-21).

Preferably, the cardiac Troponin is Troponin T, in particular human Troponin T. Preferably, the amount of Troponin T is measured by using an high sensitive Troponin assay as described in the Examples, or in WO2012/025355.

The marker Seprase is well known in the art and is e.g. described by Pineiro-Sanchez, M.L. et.al., J. Biol. Chem. 272 (1997) 7595-7601, or Park, J.E. et.al., J. Biol. Chem. 274 (1999) 36505-36512. Seprase, also known as fibroblast activation protein alpha (= FAP), or Integral membrane serine protease in the sense of the present invention, preferably is as a 170 kDa glycoprotein having gelatinase and dipeptidyl peptidase activity (EC 3.4.21.-). A comprehensive overview on the marker seprase can be assessed via Swissprot Accession Number Q12884, Version 142, last modified on February 19, 2014. Preferably, the seprase comprises two identical monomeric Seprase units (see Pineiro- Sanchez et al. and Parket al.). The monomer of the human membrane bound Seprase protein comprises 760 amino acids and is shown in UniProtKB/Swiss-Prot accession number Q12884 or in SEQ ID NO: 6 of WO2012/123293. Human Seprase is predicted to have its first 4 N-terminal residues within the fibroblast cytoplasm, followed by a 21 -residue transmembrane domain and then a 734 residue extracellular C-terminal catalytic domain (Goldstein, L.A. et al., Biochim Biophys Acta. 1361 (1997) 11-19; Scanlan, M.J. et al., Proc Natl Acad Sci USA 91 (1994) 5657-5661). A shorter form of human Seprase protein is known to a person skilled in the art as soluble Seprase or circulating antiplasmin-cleaving enzyme (= APCE) (Lee, K.N. et al., Blood 103 (2004) 3783-3788; Lee, K.N. et al., Blood 107 (2006) 1397-1404). Preferably, soluble seprase comprises the amino acid positions 26-760 of Isoform 1 from Swissprot database Accession number Q12884 (see in particular Isoform 1, last modified March 23, 2010, Version 5, Checksum: 7FF817B5A4F75142), or of the polypeptide as shown in SEQ ID NO: 6 of WO2012/123293. The Human Protein Atlas records existence of 6 isoforms for Seprase, including 2 with predicted signal peptide. Uniprot describes 2 isoforms produced by alternative splicing: canonical isoform of 760aa and truncated S isoform with aa 1-521 missing. The dimer of soluble Seprase is a 160 kDa glycoprotein consisting of two identical monomeric soluble Seprase protein units. Pineiro-Sanchez et al. (supra) found that a increased expression of Seprase correlates with the invasive phenotype of human melanoma and carcinoma cells. Henry, L.R. et al., Clin. Cancer Res. 13 (2007) 1736-1741 describe that human colon tumor patients having high levels of stromal Seprase are more likely to have aggressive disease progression and potential development of metastases or recurrence.

In an embodiment of the present invention, the term "seprase" refers to soluble seprase (see above). Accordingly, it is envisaged to measure the level of soluble seprase in a sample as specified herein, in particular the level of the dimer of soluble seprase.

The term "level" as used herein encompasses the absolute amount of a biomarker as referred to herein, the relative amount or concentration of the said biomarker as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels measured from biological read out systems in response to the peptides or intensity signals obtained from specifically bound ligands. It is to be understood that values correlating to the aforementioned levels or parameters can also be obtained by all standard mathematical operations.

The term "comparing" as used herein refers to comparing the level of the biomarker in the sample from the individual or patient with the reference level of the biomarker specified elsewhere in this description. It is to be understood that comparing as used herein usually refers to a comparison of corresponding parameters or values, e.g., an absolute amount is compared to an absolute reference amount while a concentration is compared to a reference concentration or an intensity signal obtained from the biomarker in a sample is compared to the same type of intensity signal obtained from a reference sample. The comparison may be carried out manually or computer assisted. Thus, the comparison may be carried out by a computing device (e.g., of a system disclosed herein). The value of the measured or detected level of the biomarker in the sample from the individual or patient and the reference level can be, e.g., compared to each other and the said comparison can be automatically carried out by a computer program executing an algorithm for the comparison. The computer program carrying out the said evaluation will provide the desired assessment in a suitable output format. For a computer assisted comparison, the value of the measured amount may be compared to values corresponding to suitable references which are stored in a database by a computer program. The computer program may further evaluate the result of the comparison, i.e. automatically provide the desired assessment in a suitable output format. For a computer assisted comparison, the value of the measured amount may be compared to values corresponding to suitable references which are stored in a database by a computer program. The computer program may further evaluate the result of the comparison, i.e. automatically provides the desired assessment in a suitable output format.

In accordance with the method of the present invention, the levels of the cardiac marker and the level of the biomarker seprase shall be compared to suitable reference level, i.e. the level of the cardiac marker shall be compared with a reference level for said cardiac marker, and the level of the biomarker seprase shall be compared with a reference level for said biomarker. Preferably, the reference levels for said cardiac marker and the biomarker seprase shall allow for the differentiation as set forth herein. As the skilled artisan will appreciate the reference levels for the cardiac marker and for the biomarker seprase are premeasured and set to meet routine requirements in terms of e.g. specificity and/or sensitivity. These requirements can vary, e.g. from regulatory body to regulatory body. It may for example be that assay sensitivity or specificity, respectively, has to be set to certain limits, e.g. 80%, 90%, 95% or 98%, respectively. These requirements may also be defined in terms of positive or negative predictive values. Nonetheless, based on the teaching given in the present invention it will always be possible for a skilled artisan to arrive at the reference level meeting those requirements. In one embodiment the reference level is measured in reference samples from healthy individuals. The reference level in one embodiment has been predetermined in reference samples from the disease entity to which the patient belongs, and thus from samples from patients suffering from acute shortness of breath. Preferably, the reference level of the biomarker seprase, i.e. the reference level to be applied in step c) of the method of the present invention can be derived from samples of patients suffering from acute shortness of breath, in particular from samples of patients suffering from acute shortness of breath with pulmonary disease, or from samples of patients suffering from acute shortness of breath without pulmonary disease. Also preferably, the reference level of the cardiac marker, i.e. the reference level to be applied in step d) of the method of the present invention can be derived from samples of patients suffering from acute shortness of breath, in particular from samples of patients suffering from acute shortness of breath with cardiac disease, or from samples of patients suffering from acute shortness of breath without cardiac disease.

In certain embodiments the reference level can e.g. be set to any percentage between 25% and 75% of the overall distribution of the values in a disease entity investigated. In other embodiments the reference level can e.g. be set to the median, tertiles or quartiles as measured from the overall distribution of the values in reference samples from a disease entity investigated. In one embodiment the reference level is set to the median value as determined from the overall distribution of the values in a disease entity investigated. The reference level may vary depending on various physiological parameters such as age, gender or subpopulation, as well as on the means used for the determination of the biomarkers referred to herein. In one embodiment, the reference sample is from essentially the same type of cells, tissue, organ or body fluid source as the sample from the individual or patient subjected to the method of the invention, e.g. if according to the invention blood is used as a sample to determine the level of the biomarkers in the individual, the reference level is also measured in blood or a part thereof.

As set forth herein, the reference levels may be predetermined levels. Preferably, the reference levels to be applied in steps c) and d) as are levels which allow for allocation of a patient with acute shortness of breath into a group of patients suffering from pulmonary disease and/or the group of patients suffering from cardiac disease.

The following applies as diagnostic algorithm:
Preferably, the patient suffers from pulmonary disease if the level of seprase is below the reference level, and/or the patient suffers from cardiac disease if the level of the cardiac troponin is above the reference level.

In particular, the following applies:
- a level of seprase below the reference level is indicative for a patient who suffers from pulmonary disease,
- a level of seprase above the reference level is indicative for a patient who does not suffer from pulmonary disease,
- a level of NT-proBNP or BNP above the reference level is indicative for a patient who suffers from cardiac disease, and/or
- a level of NT-proBNP or BNP below the reference level is indicative for a patient who does not suffer from cardiac disease.

Preferred reference levels to be applied in accordance with the present invention are within a range of about 39 ng/ml to about 159 ng/ml, in particular about 47 ng/ml to about 144 ng/ml for the biomarker seprase. In a preferred embodiment, the reference level for this marker is within a range of about 67 ng/ml to about 78 ng/ml. Also preferably, the reference level may be about 78 ng/ml. Further preferably, the reference level may be about 84 ng/ml.

Preferred reference levels for the marker NT-proBNP (or BNP) to be applied in accordance with the present invention are within a range of about 100 to about 500 pg/ml for NT-proBNP (about 80 to about 130 pg/ml for BNP), in particular about 250 to about 450 pg/ml for NT-proBNP (about 90 to about 110 pg/ml for BNP). In a preferred embodiment, the reference level for this NT-proBNP is about 400 pg/ml, and the reference level is about 100 pg/ml for BNP. Further, it is envisaged that the reference level is about 125 pg/ml for NT-proBNP.

In certain embodiments, the term "above the reference level" refers to a level of the biomarker in the sample from the individual or patient above the reference level or to an overall increase of 5%, 10%, 20%, 25%, 30%, 40%, 50%, 60%, 70%, 80%, 85%, 90%, 95%, 100% or greater, determined by the methods described herein, as compared to the reference level. In certain embodiments, the term increase refers to the increase in biomarker level in the sample from the individual or patient wherein, the increase is at least about 1.5-, 1.75-, 2-, 3-, 4-, 5-, 6-, 7-, 8-, 9-, 10-, 15-, 20-, 25-, 30-, 40-, 50-, 60-, 70-, 75-, 80-, 90-, or 100- fold higher as compared to the reference level, e.g. predetermined from a reference sample.

In certain embodiments, the term "decrease" or "below" herein refers to a level of the biomarker in the sample from the individual or patient below the reference level or to an overall reduction of 5%, 10%, 20%, 25%, 30%, 40%, 50%, 60%, 70%, 80%, 85%, 90%, 95%, 96%, 97%, 98%, 99% or greater, determined by the methods described herein, as compared to the reference level. In certain embodiments, the term decrease in biomarker level in the sample from the individual or patient wherein the decreased level is at most about 0.9-, 0.8-, 0.7-, 0.6-, 0.5-, 0.4-, 0.3-, 0.2-, 0.1-, 0.05-, or 0.01- fold of the reference level, e.g. predetermined from a reference sample, or lower.

Preferably, the term "about" as used herein encompasses a range of + and - 20%, more preferably a range of + and - 10%, even more preferably a range of + and - 5%, and most preferably a range of + and - 2%, relative to the specific amount, e.g., indication of a a level of "about 100" is meant to encompass a level within a range from 80 to 120. Also, the term "about" refers to the exact level. Preferably, the levels are measured as described in the Examples.

The definitions and explanations given herein above apply *mutatis mutandis* to following embodiments of the present invention.

The present invention further relates to a method for differentiating in a patient who suffers from acute shortness of breath (acute dyspnea) between (i) a pulmonary disease without cardiac disease, (ii) a cardiac disease without pulmonary disease, (iii) a cardiac disease accompanied by a pulmonary disease, and (iv) acute dyspnea without cardiac and pulmonary disease, said method comprising the steps of
a) measuring a level of seprase in a sample from said patient,
b) measuring a level of NT-proBNP or BNP in a sample from said patient,
c) comparing the level as measured in step a) to a reference level, and
d) comparing the level as measured in step b) to a reference level.

In an embodiment, the aforementioned method may comprise step e) of differentiating (or providing a differentiation) between (i), (ii), (iii) and (iv) based on the results of the comparison steps c) and d).

The term "differentiating" has been defined herein above. In accordance with the aforementioned method, the term "differentiating" in particular, means to distinguish between a subject which suffers from (i) a pulmonary disease, (ii) a cardiac disease, (iii) a cardiac disease accompanied by a pulmonary disease or (iv) acute dyspnea without cardiac and pulmonary disease (i.e. acute dyspnea without cardiac or pulmonary causes under conditions). The term in accordance with the aforementioned method, preferably, includes differentially diagnosing a pulmonary disease, a cardiac disease, a cardiac disease accompanied by a pulmonary disease or acute dyspnea without cardiac and pulmonary causes (i.e. acute shortness of breath without cardiac disease and pulmonary disease).

The terms "cardiac disease" and "pulmonary disease" have been defined above.

By "a cardiac disease accompanied by a pulmonary disease" it is meant that the subject shall suffer from a cardiac disease and from a pulmonary disease. It is to be understood that the two diseases or disorders may appear independently, i.e. without one causing the other. However, cases in which a primary cardiac disease as referred to herein causes a secondary pulmonary diseases and vice versa are also, preferably, encompassed from the above expression. Thus, the cardiac disease may be caused by said pulmonary disease and vice versa.

Further, acute dyspnea may, whatsoever, be observed in patients which neither suffer from cardiac disease nor from pulmonary disease. Such cases shall be comprised by the term "acute dyspnea without cardiovascular or pulmonary disease" for the purpose of the present invention. Preferred non-cardiac and non-pulmonary causes of acute shortness of breath are, preferably, obesity, high body weight, an untrained or poorly trained physical condition of the subject, psychological conditions such as anxiety states. Accordingly, the expression "acute dyspnea without cardiac and pulmonary disease" means that the patient suffering from acute shortness of breath suffers from a non-pulmonary and non-cardiac disease.

The term "reference level" has been defined above. The definition applies accordingly. Preferably, the reference levels to be applied in steps c) and d) are levels which allow for allocation of a patient with acute shortness of breath into a group of patients suffering i) from pulmonary disease without cardiac disease, (ii) from cardiac disease without pulmonary disease, (iii) from cardiac disease accompanied by pulmonary disease, or (iv) from acute dyspnea without cardiac and pulmonary disease.

The following preferably applies as diagnostic algorithm:
i) the patient suffers from pulmonary disease without cardiac disease if the measured level of seprase is below the reference level and the measured level of NT-proBNP or BNP is below the reference level,
ii) the patient suffers from cardiac disease without pulmonary disease if the measured level of seprase is above the reference level and the measured level of NT-proBNP or BNP is above the reference level,
iii) the patient suffers from cardiac disease accompanied by a pulmonary disease if the measured level of seprase is below the reference level and the measured level of NT-proBNP or BNP is above the reference level, and/or
iv) the patient suffers from acute dyspnea without cardiac and pulmonary disease if the measured level of seprase is above the reference level and the measured level of NT-proBNP or BNP is below the reference level.

In a preferred embodiment of the methods here disclosed, said methods further comprise the step of selecting, recommending and/or initiating a suitable therapy, depending on whether the subject suffers from cardiac disease and/or pulmonary disease.

The phrase "recommending a therapy" as used herein refers to using the information or data generated relating to the levels of the biomarkers as referred in accordance with the present invention in a sample of a patient for recommending a suitable therapy. If the patient is identified to suffer from pulmonary disease a suitable therapy is preferably a therapy that aims to treat said pulmonary disease. Such a therapy is well known in the art and may depend on the type of the underlying pulmonary disease. For example, the therapy may be administration of at least one anticholinergic, at least one beta 2-adrenergic agonist and/or at least one steroid, or oxygen therapy if the pulmonary disease is COPD or asthma, or administration of at least one antibiotic if the pulmonary disease is pneumonia, or immunosuppressive therapy if the pulmonary disease is pulmonary fibrosis.

If the patient is identified to suffer from cardiac disease a suitable therapy is preferably a therapy that aims to treat said cardiac disease. Such a therapy is well known in the art and may depend on the type of the underlying cardiac disease. For example, the therapy may be administration of aspirin, of clopidogrel, of ticagrelor, and/or of nitroglycerin, or angioplasty for an acute coronary syndrome (ACS), or administration of at least one prostacyclin analogue, of at least one endothelin receptor antagonist, and/or of at least one phosphodiesterase-5 inhibitor for pulmonary hypertension, or administration of least one diuretic and/or of at least one anticoagulant for right ventricular heart failure. The information or data used or generated may be in any form, written, oral or electronic. In some embodiments, using the information or data generated includes communicating, presenting, reporting, storing, sending, transferring, supplying, transmitting, dispensing, or combinations thereof. In some embodiments, communicating, presenting, reporting, storing, sending, transferring, supplying, transmitting, dispensing, or combinations thereof are performed by a computing device, analyzer unit or combination thereof. In some further embodiments, communicating, presenting, reporting, storing, sending, transferring, supplying, transmitting, dispensing, or combinations thereof are performed by a laboratory or medical professional. In some embodiments, the information or data includes a comparison of the levels of the biomarkers as referred to in accordance with the method of the present invention to reference levels. Also disclosed is a method of treating a subject suffering from acute shortness of breath, comprising
a) measuring a level of seprase in a sample from said patient,
b)measuring a level of a cardiac marker, in particular a natriuretic peptide or a cardiac troponin, in a sample from said patient,
c)comparing the level as measured in step a) to a reference level, and
d)comparing the level as measured in step b) to a reference level,
e) identifying a subject as being eligible to a therapy that aims to treat a pulmonary disease and/or a therapy that aims to treat a cardiac disease, in particular based on the results of the comparison steps c) and d), and
d) selecting, initiating, recommending and/or continuing a therapy that aims to treat pulmonary disease and/or a therapy that aims to treat cardiac disease.

Moreover, the present invention relates to the use of
a) the biomarker seprase and NT-proBNP or BNP and/or
b) an agent which specifically binds to the biomarker seprase and an agent which binds to NT-proBNP or BNP in a sample from a patient who suffers from acute shortness of breath (acute dyspnea) for differentiating between pulmonary disease and cardiac disease.

Further, also disclosed is the use of
a) the biomarker seprase and a cardiac marker, and/or
b) an agent which specifically binds to the biomarker seprase and an agent which binds to a cardiac marker
in a sample from a patient who suffers from acute shortness of breath (acute dyspnea) for differentiating between (i) pulmonary disease without cardiac disease, (ii) cardiac disease without pulmonary disease, (iii) cardiac disease accompanied by pulmonary disease, and (iv) acute dyspnea without cardiac and pulmonary disease.

Also disclosed is the use of
a) the biomarker seprase and a cardiac marker, and/or
b) an agent which specifically binds to the biomarker seprase and an agent which binds to a cardiac marker
for the manufacture of a diagnostics for differentiating in a patient who suffers from acute shortness of breath between pulmonary disease and cardiac disease.

In addition, also disclosed is the use of
a) the biomarker seprase and a cardiac marker, and/or
b) an agent which specifically binds to the biomarker seprase and an agent which binds to a cardiac marker
for the manufacture of a diagnostics for differentiating in a patient who suffers from acute shortness of breath between (i) pulmonary disease without cardiac disease, (ii) cardiac disease without pulmonary disease, (iii) cardiac disease accompanied by pulmonary disease, and (iv) acute dyspnea without cardiac and pulmonary disease.

In accordance with the present invention, the agent which specifically binds the biomarker seprase and the cardiac marker, respectively, preferably, shall be a detection agent which which specifically binds the biomarker seprase and the cardiac marker. The terms "agent" and "detection agent" were specified above. Preferably, the agent is an antibody which specifically binds that marker. More preferably, the antibody is a polyclonal antibody. Most preferably, the antibody is a monoclonal antibody. However, other detections agents may be applied as well.

According to a preferred embodiment of the present invention, a device adapted for carrying out a method of the invention is provided comprising
a. an analyzer unit comprising an agent which specifically binds to the biomarker seprase, and a detection agent which specifically binds a NT-proBNP or BNP, said unit being adapted for measuring the levels of the markers in a sample from a subject who suffers from acute shortness of breath; and
b. an analyzer unit (or evaluation unit) for comparing the measured levels with reference levels, whereby it is differentiated between pulmonary disease and cardiac disease, said unit comprising a database with reference levels, and an algorithm for carrying out the comparison.

Also disclosed is a device adapted for carrying out a method of the invention is provided comprising
a. an analyzer unit comprising an agent which specifically binds to the biomarker seprase, and a detection agent which specifically binds a cardiac marker, said unit being adapted for measuring the levels of the markers in a sample from a subject who suffers from acute shortness of breath; and
b. an analyzer unit (or evaluation unit) for comparing the measured levels with reference levels, whereby it is differentiated between (i) a pulmonary disease without cardiac disease, (ii) a cardiac disease without pulmonary disease, (iii) a cardiac disease accompanied by a pulmonary disease, and (iv) acute dyspnea without cardiac and pulmonary disease, said unit comprising a database with reference levels, and an algorithm for carrying out the comparison.

Preferably, the algorithm is a computer-implemented algorithm. Preferably, the algorithm is the diagnostic algorithm as set forth elsewhere herein. Preferred reference levels are disclosed elsewhere herein.

A preferred embodiment of the instant disclosure includes a system for differentiating between cardiac disease and pulmonary disease (or (i) a pulmonary disease without cardiac disease, (ii) a cardiac disease without pulmonary disease, (iii) a cardiac disease accompanied by a pulmonary disease, and (iv) acute dyspnea without cardiac and pulmonary disease) in a patient suffering from acute shortness of breath. Examples of systems include clinical chemistry analyzers, coagulation chemistry analyzers, immunochemistry analyzers, urine analyzers, nucleic acid analyzers, used to detect the result of chemical or biological reactions or to monitor the progress of chemical or biological reactions. More specifically, exemplary systems of the instant disclosure may include Roche Elecsys™ Systems and Cobas^{®} e Immunoassay Analyzers, Abbott Architect™ and Axsym™ Analyzers, Siemens Centaur™ and Immulite™ Analyzers, and Beckman Coulter UniCel™ and Acess™ Analyzers, or the like.

Embodiments of the system may include one or more analyzer units utilized for practicing the subject disclosure. The analyzer units of the system disclosed herein are in operable communication with the computing device disclosed herein through any of a wired connection, Bluetooth, LANS, or wireless signal, as are known. Additionally, according to the instant disclosure, an analyzer unit may comprise a stand-alone apparatus, or module within a larger instrument, which performs one or both of the detection, e.g. qualitative and/or quantitative evaluation of samples for diagnostic purpose. For example, an analyzer unit may perform or assist with the pipetting, dosing, mixing of samples and/or reagents. An analyzer unit may comprise a reagent holding unit for holding reagents to perform the assays. Reagents may be arranged for example in the form of containers or cassettes containing individual reagents or group of reagents, placed in appropriate receptacles or positions within a storage compartment or conveyor. Detection reagents may also be in immobilized form on a solid support which are contacted with the sample. Further, an analyzer unit may include a process and/or detection component which is optimizable for specific analysis.

According to some embodiments, an analyzer unit may be configured for optical detection of an analyte, for example a marker, with a sample. An exemplary analyzer unit configured for optical detection comprises a device configured for converting electro-magnetic energy into an electrical signal, which includes both single and multi-element or array optical detectors. According to the present disclosure, an optical detector is capable of monitoring an optical electro-magnetic signal and providing an electrical outlet signal or response signal relative to a baseline signal indicative of the presence and/or concentration of an analyte in a sample being located in an optical path. Such devices may also include, for example, photodiodes, including avalanche photodiodes, phototransistors, photoconductive detectors, linear sensor arrays, CCD detectors, CMOS detectors, including CMOS array detectors, photomultipliers, and photomultiplier arrays. According to certain embodiments, an optical detector, such as a photodiode or photomultiplier, may contain additional signal conditioning or processing electronics. For example, an optical detector may include at least one pre-amplifier, electronic filter, or integrated circuit. Suitable pre-preamplifiers include, for example, integrating, transimpedance, and current gain (current mirror) preamplifiers.

Additionally, one or more analyzer unit according to the instant disclosure may comprise a light source for emitting light. For example, a light source of an analyzer unit may consist of at least one light emitting element (such as a light emitting diode, an electric powered radiation source such as an incandescent lamp, an electroluminescent lamp, a gas discharge lamp, a high-intensity discharge lamp, a laser) for measuring analyte concentrations with a sample being tested or for enabling an energy transfer (for example, through florescent resonance energy transfer or catalyzing an enzyme).

Further, an analyzer unit of the system may include one or more incubation units (for example, for maintaining a sample or a reagent at a specified temperature or temperature range). In some embodiments, an analyzer unit may include a thermocycler, include a real-time thermocycler, for subjecting a sample to repeated temperature cycles and monitoring a change in the amount of an amplification product with the sample.

Additionally, an analyzer unit of the system disclosed herein may comprise, or be operationally connected to, a reaction vessel or cuvette feeding unit. Exemplary feeding units include liquid processing units, such as a pipetting unit, to deliver samples and/or reagents to the reaction vessels. The pipetting unit may comprise a reusable washable needle, e.g. a steel needle, or disposable pipette tips. The analyzer unit may further comprise one or more mixing units, for example a shaker to shake a cuvette comprising a liquid, or a mixing paddle to mix liquids in a cuvette, or reagent container.

It follows from the above that according to some embodiments of the instant disclosure, portions of some steps of methods disclosed and described herein may be performed by a computing device. A computing device may be a general purpose computer or a portable computing device, for example. It should also be understood that multiple computing devices may be used together, such as over a network or other methods of transferring data, for performing one or more steps of the methods disclosed herein. Exemplary computing devices include desktop computers, laptop computers, personal data assistants ("PDA"), such as BLACKBERRY brand devices, cellular devices, tablet computers, servers, and the like. In general, a computing device comprises a processor capable of executing a plurality of instructions (such as a program of software).

A computing device has access to a memory. A memory is a computer readable medium and may comprise a single storage device or multiple storage devices, located either locally with the computing device or accessible to the computing device across a network, for example. Computer-readable media may be any available media that can be accessed by the computing device and includes both volatile and non-volatile media. Further, computer readable-media may be one or both of removable and non-removable media. By way of example, and not limitation, computer-readable media may comprise computer storage media. Exemplary computer storage media includes, but is not limited to, RAM, ROM, EEPROM, flash memory or any other memory technology, CD-ROM, Digital Versatile Disk (DVD) or other optical disk storage, magnetic cassettes, magnetic tape, magnetic disk storage or other magnetic storage devices, or any other medium which can be used for storing a plurality of instructions capable of being accessed by the computing device and executed by the processor of the computing device.

According to embodiments of the instant disclosure, software may include instructions which, when executed by a processor of the computing device, may perform one or more steps of the methods disclosed herein. Some of the instructions may be adapted to produce signals that control operation of other machines and thus may operate through those control signals to transform materials far removed from the computer itself. These descriptions and representations are the means used by those skilled in the art of data processing, for example, to most effectively convey the substance of their work to others skilled in the art.

The plurality of instructions may also comprise an algorithm which is generally conceived to be a self-consistent sequence of steps leading to a desired result. These steps are those requiring physical manipulations of physical quantities. Usually, though not necessarily, these quantities take the form of electrical or magnetic pulses or signals capable of being stored, transferred, transformed, combined, compared, and otherwise manipulated. It proves convenient at times, principally for reasons of common usage, to refer to these signals as values, characters, display data, numbers, or the like as a reference to the physical items or manifestations in which such signals are embodied or expressed. It should be borne in mind, however, that all of these and similar terms are to be associated with the appropriate physical quantities and are merely used here as convenient labels applied to these quantities. According to some embodiments of the instant disclosure, an algorithm for carrying out a comparison between a measured amount of one or more markers disclosed herein, and a suitable reference, is embodied and performed by executing the instructions. The results may be given as output of parametric diagnostic raw data or as absolute or relative amounts. According to various embodiments of the system disclosed herein, a "diagnosis" may be provided by the computing device of a system disclosed herein based on said comparison of the calculated "amount" to a reference or a threshold. For example, a computing device of a system may provide an indicator, in the form of a word, symbol, or numerical value which is indicative of a particular diagnosis.

The computing device may also have access to an output device. Exemplary output devices include fax machines, displays, printers, and files, for example. According to some embodiments of the present disclosure, a computing device may perform one or more steps of a method disclosed herein, and thereafter provide an output, via an output device, relating to a result, indication, ratio or other factor of the method.

Finally, the invention pertains to a kit preferably adapted for carrying out a method of the present invention comprising an agent which specifically binds to the biomarker seprase, and an agent which specifically binds to NT-proBNP or BNP. Preferably, the kit further comprises reference standards for the biomarker seprase and/or the cardiac marker as well as instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Further, the kit shall comprise at least one standard for a reference as defined herein above, i.e. a solution with a pre-defined level (levels) for the cardiac marker and/or the biomarker seprase representing a reference level (or reference levels) as set for th elsewhere herein.

In some embodiments, a kit disclosed herein includes at least one component or a packaged combination of components for practicing a disclosed method. By "packaged combination" it is meant that the kits provide a single package that contains a combination of one or more components, such as probes (for example, an antibody), controls, buffers, reagents (for example, conjugate and/or substrate) instructions, and the like, as disclosed herein. A kit containing a single container is also included within the definition of "packaged combination." In some embodiments, the kits include at least one probe, for example an antibody (having specific affinity for an epitope of a biomarker as disclosed herein. For example, the kits may include an antibody that is labelled with a fluorophore or an antibody that is a member of a fusion protein. In the kit, the probe may be immobilized, and may be immobilised in a specific conformation. For example, an immobilized probe may be provided in a kit to specifically bind target protein, to detect target protein in a sample, and/or to remove target protein from a sample.

According to some embodiments, kits include at least one probe, which may be immobilized, in at least one container. Kits may also include multiple probes, optionally immobilized, in one or more containers. For example, the multiple probes may be present in a single container or in separate containers, for example, wherein each container contains a single probe.

In some embodiments, a kit may include one or more non-immobilized probe and one or more solid support that does or does not include an immobilized probe. Some such embodiments may comprise some or all of the reagents and supplies needed for immobilizing one or more probes to the solid support, or some or all of the reagents and supplies needed for binding of immobilized probes to specific proteins within a sample.

In certain embodiments, a single probe (including multiple copies of the same probe) may be immobilized on a single solid support and provided in a single container. In other embodiments, two or more probes, each specific for a different target protein or a different form of a single target protein (such as a specific epitope), a provided in a single container. In some such embodiments, an immobilized probe may be provided in multiple different containers (e.g., in single-use form), or multiple immobilized probes may be provided in multiple different containers. In further embodiments, the probes may be immobilized on multiple different type of solid supports. Any combination of immobilized probe(s) and container(s) is contemplated for the kits disclosed herein, and any combination thereof may be selected to achieve a suitable kit for a desired use.

A container of the kits may be any container that is suitable for packaging and/or containing one or more components disclosed herein, including for example probes (for example, an antibody), controls, buffers, and reagents (for example, conjugate and/or substrate). Suitable materials include, but are not limited to, glass, plastic, cardboard or other paper product, wood, metal, and any alloy thereof. In some embodiments, the container may completely encase an immobilized probe(s) or may simply cover the probe to minimize contamination by dust, oils, etc., and expose to light. In some further embodiments, he kits may comprise a single container or multiple containers, and where multiple containers are present, each container may be the same as all other containers, different than others, or different than some but not all other containers.

The Figures show:

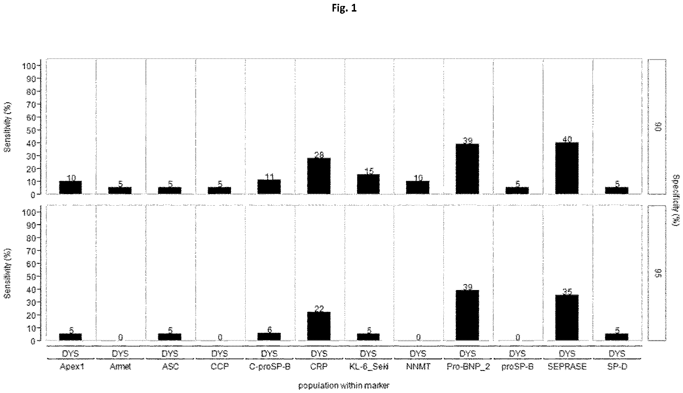

**Fig. 1****:** Dyspnea subgroups pulmonal *versus* cardial. Differential diagnostic accuracy of various biomarkers (Seprase, NT-proBNP, SP-B, proSP-B, C-terminal proSP-B, CRP (C-reactive protein) APEX1 (DNA-(apurinic or apyrimidinic site) lyase), ARMET (arginine-rich metastasized in early tumors protein), CCP (cyclic citrullinated peptide), ASC (apop-tosis-associated speck-like protein), KL-6, NNMT (nicotinamide N-methyltransferase)). The figure shows barplots for the sensitivity at a specificity of 90% and 95%, respectively. As compared to the other markers, NT-proBNP and Seprase conferred a suitable sensitivity (Subgroup "cardial" regarded as healthy for calculation of specificity. Only for NT-proBNP, subgroup pulmonal regarded as healthy).

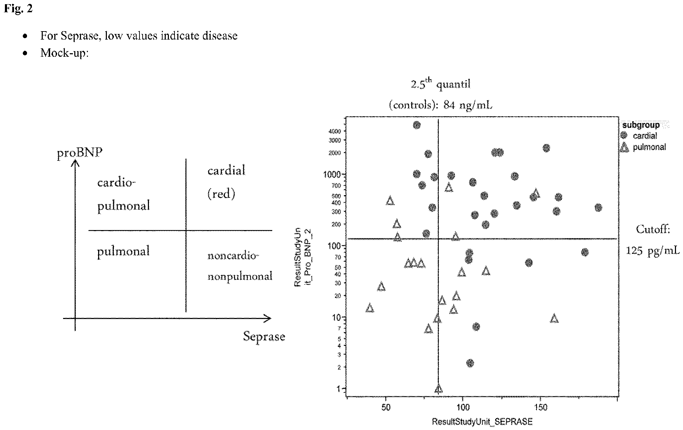

**Fig. 2****:** Dyspnea subgroups pulmonal, cardial, cardio-pulmonal (model) and noncardiononpulmonal (model); scatter plot for seprase and proBNP values in four abovementioned diagnoses (see (i), (ii), (iii) and (iv) as referred to above). Pulmonal disease is indicated by seprase values below cutoff of 84 ng/ml, cardial dyspnea is indicated by proBNP values above cutoff of 125 pg/ml.

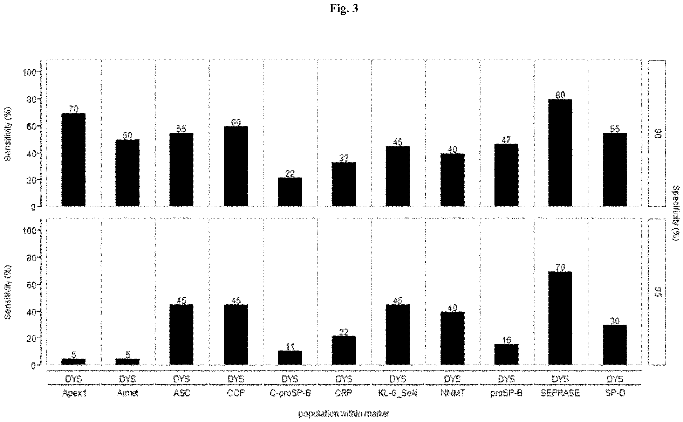

**Fig. 3****:** Sensitivity to diagnose pulmonal disease for each biomarker combined with proBNP. (Seprase, NT-proBNP, SP-B, proSP-B, C-terminal proSP-B, CRP (C-reactive protein) APEX1 (DNA-(apurinic or apyrimidinic site) lyase), ARMET (arginine-rich metastasized in early tumors protein), CCP (cyclic citrullinated peptide), ASC (apoptosis-associated speck-like protein), KL-6, NNMT (nicotinamide N-methyltransferase)). The figure shows barplots for each marker sensitivity at a specificity of 90% and 95%, respectively, with cardial cohort defined as control. Improvement of diagnostic sensitivity by combination with proBNP was observed only for seprase (80% and 70%, sensitivity respectively, proBNP alone: Sensitivity 39%).

### EXAMPLES

The invention will now be illustrated by the following Examples which are not intended to restrict or limit the scope of this invention.

### Example 1: Assays

Cystatin C was measured by using an immunoturbidimetric assay for the quantitative in vitro determination of cystatin C in human serum and plasma on Roche automated clinical chemistry analyzers (Assay: Tina-quant Cystatin C, Roche Diagnostics GmbH, Mannheim, Germany). In this assay human cystatin C agglutinates with latex particles coated with anti-cystatin C antibodies. The aggregate is measured turbidimetrically at 546 nm.

NT-proBNP was measured using Roche's electrochemiluminescence ELISA sandwich test Elecsys proBNP II STAT (Short Turn Around Time) assay. The test employs two monoclonal antibodies which recognize epitopes located in the N-terminal part (1-76) of proBNP (1-108).

Seprase was measured in serum samples using monoclonal anti seprase antibodies generated in mouse. For the generation of a monoclonal antibody eight week old female Balb/c mice were immunized intraperitoneally with 30 µg recombinant extracellular (aa 26- 760) seprase fragment expressed in HEK293 expression system recombinant seprase. The initial immunization was followed by three further immunizations with iFA after 6 weeks at monthly intervals. Three days before spleen removal mice were boosted intravenously with 5 µg of the antigen. Spleen cells and P3X63Ag8.653 myeloma cells were fused with PEG and cultured in HAz selection medium. After removal, single cell preparations of the spleen were made and spleen cells were fused to myeloma cells as e.g. described in Köhler and Milstein (1975). Resulting hybridomas were screened with ELISA for reactivity against a biotinylated antigen. Hybridomas with high affinity for antigen were cloned by single cell deposition using a FACSAria III. The resulting monoclonal subclones were again tested by ELISAMonoclonal IgG were subsequently biotinylated and digoxygenylated as described in patent application WO2012/123293.

### Example 2: Patient cohort/Results

Serum samples from patients with acute dyspnea were examined, n=28 had a cardiac cause and n=20 a pulmonic cause.

In the dyspnea cohort the clinical sensitivity was calculated at 90% and 95% specificity, with subgroup cardiac (n =28) defined as "healthy" for calculation of specificity. Evaluation of the Seprase assay showed 40% and 35% sensitivity at 90% and 95% specificity, respectively. In comparison, diagnostic accuracy of NT-proBNP test (with subgroup pulmonary, n= 20, regarded as "healthy"), was 39.3 % sensitivity for both 90% and 95% specificity (Figure 1). As high values for NT-proBNP indicate cardiac cause whereas low values for Seprase are supposed to indicate pulmonary cause of dyspnea, patients with clinically diagnosed mere cardiac cause of dyspnea would have higher values for both assays, whereas patients with clinically diagnosed mere pulmonary cause would have lower values on both (see mock-up in Fig. 2). Fig. 2 shows that this relation is true for the majority of patients. Combination of Seprase and NT-proBNP notably improved sensitivity of Seprase test, resulting in sensitivity of 80% (specificity 90%) or 70% (specificity 95%; Figure 3).

**Conclusion:** Seprase could be used a marker of choice for differentiation of pulmonary and cardiac dyspnea. Application of Seprase alone or in combination with NT-proBNP or with other cardiac markers could considerably reduce the need for echocardiographic screening and improve the evaluation and treatment of patients with acute dyspnea.

## Claims

1. A method for differentiating in a patient who suffers from acute shortness of breath (acute dyspnea) between pulmonary disease and cardiac disease, said method comprising the steps of
a) measuring a level of seprase in a sample from said patient,
b) measuring a level of NT-proBNP or a level of BNP in a sample from said patient,
c) comparing the level as measured in step a) to a reference level, and
d) comparing the level as measured in step b) to a reference level.

2. The method according to claim 1, wherein the patient suffers from pulmonary disease if the level of seprase is below the reference level, and/or wherein the patient suffers from cardiac disease if the level of NT-proBNP or BNP is above the reference level.

3. The method of claims 1 and 2, wherein the patient is a human patient.

4. The method of any one of claims 1 to 3, wherein the sample is a blood, serum or plasma sample.

5. A method for differentiating in a patient who suffers from acute shortness of breath (acute dyspnea) between (i) a pulmonary disease without cardiac disease, (ii) a cardiac disease without pulmonary disease, (iii) a cardiac disease accompanied by a pulmonary disease, and (iv) acute dyspnea without cardiac and pulmonary disease, said method comprising the steps of
a) measuring a level of seprase in a sample from said patient,
b) measuring a level of a NT-proBNP or a level of BNP in a sample from said patient,
c) comparing the level as measured in step a) to a reference level, and
d) comparing the level as measured in step b) to a reference level.

6. The method of claim 5, wherein the patient is a human patient.

7. The method of claims 5 and 6, wherein
i) the patient suffers from pulmonary disease without cardiac disease if the measured level of seprase is below the reference level and the measured level of NT-proBNP or BNP is below the reference level,
ii) the patient suffers from cardiac disease without pulmonary disease if the measured level of seprase is above the reference level and the measured level of NT-proBNP or BNP is above the reference level,
iii) the patient suffers from cardiac disease accompanied by a pulmonary disease if the measured level of seprase is below the reference level and the measured level of NT-proBNP or BNP is above the reference level, and/or
iv) the patient suffers from acute dyspnea without cardiac and pulmonary disease if the measured level of seprase is above the reference level and the measured level of NT-proBNP or BNP is below the reference level.

8. Use of
a) the biomarker i) seprase and ii) the biomarker NT-proBNP or the biomarker BNP, and/or
b) an antibody or antibody fragment which specifically binds to the biomarker seprase, and an antibody or antibody fragment which binds to NT-proBNP or BNP
in a sample of a subject patient who suffers from acute shortness of breath for differentiating between pulmonary disease and cardiac disease.

9. A device adapted for carrying out a method according to any one of claims 1 to 5, comprising
a. an analyzer unit comprising an antibody or antibody fragment which specifically binds to the biomarker seprase, and an antibody or antibody fragment which specifically binds NT-proBNP or BNP, said unit being adapted for measuring the levels of the markers in a sample from a subject who suffers from acute shortness of breath; and
b. an analyzer unit (or evaluation unit) for comparing the measured levels with reference levels, whereby it is differentiated between pulmonary disease and cardiac disease, said unit comprising a database with reference levels, and an algorithm for carrying out the comparison.

10. A kit comprising an antibody or antibody fragment which specifically binds to the biomarker seprase, and an antibody or antibody fragment which specifically binds to NT-proBNP or BNP.

## Patentansprüche

1. Verfahren zum Unterscheiden zwischen Lungenerkrankung und Herzerkrankung bei einem Patienten, der an akuter Atemnot (akuter Dyspnoe) leidet, wobei das Verfahren die Schritte umfasst:
a) Messen der Menge von Seprase in einer Probe des Patienten,
b) Messen der Menge von NT-proBNP oder der Menge von BNP in einer Probe des Patienten,
c) Vergleichen der bei Schritt a) gemessenen Menge mit einer Referenzmenge und
d) Vergleichen der bei Schritt b) gemessenen Menge mit einer Referenzmenge.

2. Verfahren gemäß Anspruch 1, wobei der Patient an Lungenerkrankung leidet, wenn die Menge von Seprase unter der Referenzmenge liegt, und/oder wobei der Patient an Herzerkrankung leidet, wenn die Menge von NT-proBNP oder BNP über der Referenzmenge liegt.

3. Verfahren gemäß Ansprüchen 1 und 2, wobei der Patient ein menschlicher Patient ist.

4. Verfahren gemäß einem der Ansprüche 1 bis 3, wobei die Probe eine Blut-, Serum- oder Plasmaprobe ist.

5. Verfahren zum Unterscheiden zwischen (i) einer Lungenerkrankung ohne Herzerkrankung, (ii) einer Herzerkrankung ohne Lungenerkrankung, (iii) einer Herzerkrankung begleitet von einer Lungenerkrankung und (iv) akuter Dyspnoe ohne Herz- und Lungenerkrankung bei einem Patienten, der an akuter Atemnot (akuter Dyspnoe) leidet, wobei das Verfahren die Schritte umfasst:
a) Messen der Menge von Seprase in einer Probe des Patienten,
b) Messen der Menge von NT-proBNP oder der Menge von BNP in einer Probe des Patienten,
c) Vergleichen der bei Schritt a) gemessenen Menge mit einer Referenzmenge und
d) Vergleichen der bei Schritt b) gemessenen Menge mit einer Referenzmenge.

6. Verfahren gemäß Anspruch 5, wobei der Patient ein menschlicher Patient ist.

7. Verfahren gemäß Ansprüchen 5 und 6, wobei
i) der Patient an Lungenerkrankung ohne Herzerkrankung leidet, wenn die gemessene Menge von Seprase unter der Referenzmenge liegt und die gemessene Menge von NT-proBNP oder BNP unter der Referenzmenge liegt,
ii) der Patient an Herzerkrankung ohne Lungenerkrankung leidet, wenn die gemessene Menge von Seprase über der Referenzmenge liegt und die gemessene Menge von NT-proBNP oder BNP über der Referenzmenge liegt,
iii) der Patient an Herzerkrankung begleitet von einer Lungenerkrankung leidet, wenn die gemessene Menge von Seprase unter der Referenzmenge liegt und die gemessene Menge von NT-proBNP oder BNP über der Referenzmenge liegt, und/oder
iv) der Patient an akuter Dyspnoe ohne Herz- und Lungenerkrankung leidet, wenn die gemessene Menge von Seprase über der Referenzmenge liegt und die gemessene Menge von NT-proBNP oder BNP unter der Referenzmenge liegt.

8. Verwendung von
a) i) dem Biomarker Seprase und ii) dem Biomarker NT-proBNP oder dem Biomarker BNP und/oder
b) einem Antikörper oder Antikörperfragment, der/das spezifisch an den Biomarker Seprase bindet, und einem Antikörper oder Antikörperfragment, der/das an NT-proBNP oder BNP bindet,
in einer Probe eines Patienten, der an akuter Atemnot leidet, zum Unterscheiden zwischen Lungenerkrankung und Herzerkrankung.

9. Vorrichtung, ausgelegt zum Durchführen eines Verfahrens gemäß einem der Ansprüche 1 bis 5, umfassend
a. eine Analyseeinheit, umfassend einen Antikörper oder ein Antikörperfragment, der/das spezifisch an den Biomarker Seprase bindet, und einen Antikörper oder ein Antikörperfragment, der/das spezifisch an NT-proBNP oder BNP bindet, wobei die Einheit dafür ausgelegt ist, die Mengen der Marker in einer Probe eines Subjekts, das an akuter Atemnot leidet, zu messen; und
b. eine Analyseeinheit (oder Auswertungseinheit) zum Vergleichen der gemessenen Mengen mit Referenzmengen, wodurch zwischen Lungenerkrankung und Herzerkrankung unterschieden wird, wobei die Einheit eine Datenbank mit Referenzwerten und einen Algorithmus zum Durchführen des Vergleichs umfasst.

10. Kit, umfassend einen Antikörper oder ein Antikörperfragment, der/das spezifisch an den Biomarker Seprase bindet, und einen Antikörper oder ein Antikörperfragment, der/das spezifisch an NT-proBNP oder BNP bindet.

## Revendications

1. Méthode de différenciation chez un patient qui souffre de dyspnée aiguë entre une maladie pulmonaire et une maladie cardiaque, ladite méthode comprenant les étapes suivantes :
a) mesure d'un niveau de séprase dans un échantillon issu dudit patient,
b) mesure d'un niveau de NT-proBNP ou d'un niveau de BNP dans un échantillon issu dudit patient,
c) comparaison du niveau mesuré dans l'étape a) à un niveau de référence, et
d) comparaison du niveau mesuré dans l'étape b) à un niveau de référence.

2. Méthode selon la revendication 1, où le patient souffre d'une maladie pulmonaire si le niveau de séprase est inférieur au niveau de référence, et/ou où le patient souffre d'une maladie cardiaque si le niveau de NT-proBNP ou de BNP est supérieur au niveau de référence.

3. Méthode selon les revendications 1 et 2, où le patient est un patient humain.

4. Méthode selon l'une quelconque des revendications 1 à 3, où l'échantillon est un échantillon de sang, de sérum ou de plasma.

5. Méthode de différenciation, chez un patient souffrant de dyspnée aiguë, entre (i) une maladie pulmonaire sans maladie cardiaque, (ii) une maladie cardiaque sans maladie pulmonaire, (iii) une maladie cardiaque accompagnée d'une maladie pulmonaire, et (iv) une dyspnée aiguë sans maladie cardiaque ni pulmonaire, ladite méthode comprenant les étapes suivantes :
a) mesure d'un niveau de séprase dans un échantillon issu dudit patient,
b) mesure d'un niveau de NT-proBNP ou d'un niveau de BNP dans un échantillon issu dudit patient,
c) comparaison du niveau mesuré dans l'étape a) à un niveau de référence, et
d) comparaison du niveau mesuré dans l'étape b) à un niveau de référence.

6. Méthode selon la revendication 5, où le patient est un patient humain.

7. Procédé selon les revendications 5 et 6, où i) le patient souffre d'une maladie pulmonaire sans maladie cardiaque si le niveau mesuré de séprase est inférieur au niveau de référence et que le niveau mesuré de NT-proBNP ou de BNP est inférieur au niveau de référence,
ii) le patient souffre d'une maladie cardiaque sans maladie pulmonaire si le niveau mesuré de séprase est supérieur au niveau de référence et que le niveau mesuré de NT-proBNP ou de BNP est supérieur au niveau de référence,
iii) le patient souffre d'une maladie cardiaque accompagnée d'une maladie cardiaque si le niveau mesuré de séprase est inférieur au niveau de référence et que le niveau mesuré de NT-proBNP ou de BNP est supérieur au niveau de référence, et/ou
iv) le patient souffre d'une dyspnée aiguë sans maladie cardiaque ni pulmonaire si le niveau mesuré de séprase est supérieur au niveau de référence et que le niveau mesuré de NT-proBNP ou de BNP est inférieur au niveau de référence.

8. Utilisation
a) du biomarqueur i) séprase et ii) du biomarqueur NT-proBNP ou du biomarqueur BNP, et/ou
b) d'un anticorps ou fragment d'anticorps qui se lie spécifiquement au biomarqueur séprase, et d'un anticorps ou fragment d'anticorps qui se lie à NT-proBNP ou BNP
dans un échantillon d'un patient sujet qui souffre de dyspnée aiguë pour la différenciation entre maladie pulmonaire et maladie cardiaque.

9. Dispositif adapté à la mise en oeuvre d'une méthode selon l'une quelconque des revendications 1 à 5, comprenant
a. une unité d'analyseur comprenant un anticorps ou fragment d'anticorps qui se lie spécifiquement au biomarqueur séprase, et un anticorps ou fragment d'anticorps qui se lie spécifiquement à NT-proBNP ou BNP, ladite unité étant adaptée à la mesure du niveau des marqueurs dans un échantillon issu d'un sujet souffrant de dyspnée aiguë ; et
b. une unité d'analyseur (ou unité d'évaluation) permettant de comparer les niveaux mesurés à des niveaux de référence, ce qui permet de différencier maladie pulmonaire et maladie cardiaque, ladite unité comprenant une base de données avec des niveaux de référence, et un algorithme permettant de mettre en oeuvre la comparaison.

10. Kit comprenant un anticorps ou fragment d'anticorps qui se lie spécifiquement au biomarqueur séprase, et un anticorps ou fragment d'anticorps qui se lie spécifiquement à NT-proBNP ou BNP.
